# EUROPEAN PATENT APPLICATION

(11) **EP 4 194 556 A1**
(43) Date of publication of application: **14.06.2023**
(21) Application number: 21853227.3
(22) Date of filing: 19.01.2021
(51) Int. Cl.: C12N 15/62, C12N 15/864, A61K 48/00, A61K 38/18, A61K 38/44, A61P 27/02, A61P 9/10

(54) **FUSION PROTEIN AND USE THEREOF**

(30) Priority: 07.08.2020 CN 202010790496
(71) Applicant: Chigenovo Co., Ltd., Beijing 102206 (CN)
(72) Inventor: CHEN, Shaohong, Beijing 102206 (CN); SHI, Tianyong, Beijing 102206 (CN); HAO, Dandan, Beijing 102206 (CN); HE, Saichao, Beijing 102206 (CN); NIU, Linlin, Beijing 102206 (CN)
(74) Representative: De Clercq & Partners
(86) International application number: PCT/CN2021/072743
(87) International publication number: WO 2022/027933

(57) **Abstract**

The present application relates to a fusion protein and use thereof. The fusion protein comprises RdCVF and a signal peptide, wherein the signal peptide is an amino acid sequence set forth in any one of SEQ ID NOs: 27-31 and 33. The present application also relates to an isolated nucleic acid molecule comprising a) a polynucleotide encoding RdCVF and b) a polynucleotide encoding a signal peptide. The present application also provides a vector, a cell, and a pharmaceutical composition containing the nucleic acid molecule, as well as medical use thereof. The fusion protein and/or isolated nucleic acid molecule described herein can effectively increase the secretion of RdCVF.

## Description

### TECHNICAL FIELD

The present application relates to the field of biomedicine, in particular to a fusion protein and use thereof.

### BACKGROUND

Bietti's crystalline dystrophy (BCD) is a rare disease of retinal degeneration, and the symptoms mainly include crystals (transparent coverings) in the cornea; small, yellow or white, crystalline deposits deposited in the photosensitive tissues of the retina; and progressive atrophy of the retina, choriocapillary, and choroid.

RdCVF is a splicing variant of the nucleoredoxin-like 1 (Nxnl1) gene, and another splicing product of this gene is RdCVFL. Mice deficient in the Nxnl1 gene can exhibit age-dependent losses of rod and cone functions and cone degeneration, as well as rod and cone hypersensitivity to oxidative stress.

### SUMMARY

The present application provides a fusion protein comprising RdCVF and a signal peptide, wherein the signal peptide is an amino acid sequence set forth in any one of SEQ ID NOs: 27-31 and 33. The present application provides an isolated nucleic acid molecule comprising a polynucleotide encoding RdCVF and a polynucleotide encoding a signal peptide. The present application also provides a vector, a cell, and a pharmaceutical composition, comprising the isolated nucleic acid molecule, as well as uses thereof. The present application also provides a method of increasing the expression level of RdCVF. The expression level and/or secretion amount of the fusion protein described herein in cells is significantly improved. The nucleic acid molecule described herein has at least the following advantages: 1) significantly increasing the expression level of RdCVF; 2) significantly improving the ability of the cell containing the isolated nucleic acid molecule to secrete RdCVF; 3) significantly enhancing the retinal function of the subject (such as animal model) administrated with the nucleic acid molecule (such as significantly increasing the amplitude of electroretinogram); and 4) significantly enhancing the function of retinal photoreceptor cells in the subject (such as animal model) administrated with the nucleic acid molecule (for example, significantly enhancing the maintenance function of retinal photoreceptor cells).

In one aspect, the present application provides a fusion protein comprising RdCVF and a signal peptide, wherein the signal peptide is an amino acid sequence set forth in any one of SEQ ID NOs: 27-31 and 33.

In certain embodiments, the RdCVF is human RdCVF.

In certain embodiments, the RdCVF comprises an amino acid sequence set forth in SEQ ID NO: 34.

In certain embodiments, the C-terminus of the signal peptide is directly or indirectly linked to the N-terminus of RdCVF.

In certain embodiments, the fusion protein further comprises a second protein different from RdCVF.

In certain embodiments, the C-terminus of the second protein is directly or indirectly linked to the N-terminus of the signal peptide.

In certain embodiments, the fusion protein sequentially comprises, from N-terminus to C-terminus, the second protein, the signal peptide, and RdCVF.

In certain embodiments, the second protein comprises CYP4V2.

In certain embodiments, the CYP4V2 comprises an amino acid sequence set forth in SEQ ID NO: 35.

In certain embodiments, the fusion protein comprises an amino acid sequence set forth in any one of SEQ ID NOs: 48-52, 54, and 55.

In another aspect, the present application provides an isolated nucleic acid molecule comprising a) a polynucleotide encoding RdCVF and b) a polynucleotide encoding the signal peptide.

In certain embodiments, the RdCVF is human RdCVF.

In certain embodiments, the RdCVF comprises an amino acid sequence set forth in SEQ ID NO: 34.

In certain embodiments, the polynucleotide encoding RdCVF comprises a nucleotide sequence set forth in SEQ ID NO: 22.

In certain embodiments, the polynucleotide encoding the signal peptide is located at 5' end of the polynucleotide encoding RdCVF and operably linked to the polynucleotide encoding RdCVF.

In certain embodiments, the 3' end of the polynucleotide encoding the signal peptide is linked to the 5' end of the polynucleotide encoding RdCVF.

In certain embodiments, the type of the signal peptide includes Sec/SPI, Sec/SPII, and/or Tat/SPI.

In certain embodiments, the signal peptide has at least 9 amino acids in length.

In certain embodiments, the signal peptide comprises an amino acid sequence set forth in any one of SEQ ID NOs: 27-31 and 33.

In certain embodiments, the polynucleotide encoding the signal peptide comprises a nucleotide sequence set forth in any one of SEQ ID NOs: 15-19 and 21.

In certain embodiments, the isolated nucleic acid molecule comprises a promoter.

In certain embodiments, the promoter is located at 5' end of the polynucleotide encoding RdCVF and operably linked to the polynucleotide encoding RdCVF.

In certain embodiments, the promoter is located at 5' end of the polynucleotide encoding the signal peptide and operably linked to the polynucleotide encoding the signal peptide.

In certain embodiments, the 3' end of the promoter is linked to the 5' end of the polynucleotide encoding the signal peptide.

In certain embodiments, the promoter comprises a nucleotide sequence set forth in SEQ ID NO: 23.

In certain embodiments, the isolated nucleic acid molecule comprises a polyadenylation (PolyA) signal site.

In certain embodiments, the PolyA signal site is located at 3' end of the polynucleotide encoding RdCVF and operably linked to the polynucleotide encoding RdCVF.

In certain embodiments, the 3' end of the polynucleotide encoding RdCVF is linked to the 5' end of the PolyA signal site.

In certain embodiments, the PolyA signal site comprises a nucleotide sequence set forth in SEQ ID NO: 25.

In certain embodiments, the isolated nucleic acid molecule sequentially comprises, in 5' to 3' direction: the promoter, the polynucleotide encoding the signal peptide, the polynucleotide encoding RdCVF, and the PolyA signal site.

In certain embodiments, the isolated nucleic acid molecule comprises a second polynucleotide encoding a second protein, wherein the second protein is different from RdCVF.

In certain embodiments, the second polynucleotide and the polynucleotide encoding RdCVF are located in the same coding sequence (CDS) region.

In certain embodiments, the isolated nucleic acid molecule comprises a polynucleotide encoding a self-cleaving peptide.

In certain embodiments, the polynucleotide encoding the self-cleaving peptide is located between the second polynucleotide and the polynucleotide encoding RdCVF.

In certain embodiments, the self-cleaving peptide comprises P2A.

In certain embodiments, the polynucleotide encoding the self-cleaving peptide comprises a nucleotide sequence set forth in SEQ ID NO: 26.

In certain embodiments, the isolated nucleic acid molecule sequentially comprises, in 5' to 3' direction: the promoter, the second polynucleotide, the polynucleotide encoding the self-cleaving peptide, the polynucleotide encoding the signal peptide, the polynucleotide encoding RdCVF, and the PolyA signal site.

In certain embodiments, the isolated nucleic acid molecule sequentially comprises, in 5' to 3' direction: the promoter, the polynucleotide encoding RdCVF, the polynucleotide encoding the self-cleaving peptide, the polynucleotide encoding the signal peptide, the second polynucleotide, and the PolyA signal site.

In certain embodiments, the second polynucleotide and the polynucleotide encoding RdCVF are located in different coding sequence regions.

In certain embodiments, the isolated nucleic acid molecule comprises at least two promoters.

In certain embodiments, the isolated nucleic acid molecule comprises at least two PolyA signal sites.

In certain embodiments, the isolated nucleic acid molecule sequentially comprises, in 5' to 3' direction: the promoter, the second polynucleotide, the PolyA signal site, the promoter, the polynucleotide encoding the signal peptide, the polynucleotide encoding RdCVF, and the PolyA signal site.

In certain embodiments, the isolated nucleic acid molecule sequentially comprises, in 5' to 3' direction: the promoter, the polynucleotide encoding RdCVF, the PolyA signal site, the promoter, the polynucleotide encoding the signal peptide, the second polynucleotide, and the PolyA signal site.

In certain embodiments, the second protein comprises CYP4V2.

In certain embodiments, the CYP4V2 comprises an amino acid sequence set forth in SEQ ID NO: 35.

In certain embodiments, the second polynucleotide comprises a polynucleotide encoding CYP4V2.

In certain embodiments, the isolated nucleic acid molecule comprises a nucleotide sequence set forth in any one of SEQ ID NOs: 37-41, 43, 45, and 47.

In another aspect, the present application provides a vector comprising the isolated nucleic acid molecule described herein.

In certain embodiments, the vector comprises a viral vector.

In certain embodiments, the vector is a viral vector, and the viral vector comprises an AAV vector.

In another aspect, the present application provides a cell comprising the isolated nucleic acid molecule described herein or the vector described herein.

In another aspect, the present application provides a pharmaceutical composition comprising the isolated nucleic acid molecule described herein, the vector described herein, and/or the cell described herein, as well as the pharmaceutically acceptable carrier.

In another aspect, the present application provides the use of the isolated nucleic acid molecule described herein, the vector described herein, and/or the cell described herein in the manufacture of a medicament for treating, alleviating, and/or preventing a disease or disorder associated with retinal pigment epithelium (RPE) atrophy.

In certain embodiments, the disease or disorder comprises Bietti's crystalline dystrophy.

In another aspect, the present application provides a method of increasing the expression level of RdCVF, comprising a step of making a nucleic acid molecule capable of encoding RdCVF to contain a polynucleotide encoding a signal peptide.

In certain embodiments, the method comprises an *in vitro* method and/or an *in vivo* method.

In certain embodiments, compared with the nucleic acid molecule without the polynucleotide encoding the signal peptide, the expression level of RdCVF secreted by the cell having the nucleic acid molecule comprising the polynucleotide encoding the signal peptide is significantly increased.

In certain embodiments, the cell comprises a mammalian cell.

In certain embodiments, the RdCVF is human RdCVF.

In certain embodiments, the RdCVF comprises an amino acid sequence set forth in SEQ ID NO: 34.

In certain embodiments, the signal peptide comprises an amino acid sequence set forth in any one of SEQ ID NOs: 27-31 and 33.

In certain embodiments, the nucleic acid molecule capable of encoding RdCVF comprises a polynucleotide encoding RdCVF, and the polynucleotide encoding RdCVF comprises a nucleotide sequence set forth in SEQ ID NO: 22.

In certain embodiments, the polynucleotide encoding the signal peptide is located at 5' end of the polynucleotide encoding RdCVF and operably linked to the polynucleotide encoding RdCVF in the nucleic acid molecule encoding RdCVF.

In certain embodiments, the 3' end of the polynucleotide encoding the signal peptide is linked to the 5' end of the polynucleotide encoding RdCVF.

In certain embodiments, the type of the signal peptide includes Sec/SPI, Sec/SPII, and/or Tat/SPI.

In certain embodiments, the signal peptide has at least 9 amino acids in length.

In certain embodiments, the signal peptide comprises a nucleotide sequence set forth in any one of SEQ ID NOs: 15-19 and 21.

In certain embodiments, the nucleic acid molecule capable of encoding RdCVF comprises a promoter.

In certain embodiments, the promoter is located at 5' end of the polynucleotide encoding RdCVF and operably linked to the polynucleotide encoding RdCVF.

In certain embodiments, the promoter is located at 5' end of the polynucleotide encoding the signal peptide and operably linked to the polynucleotide encoding the signal peptide.

In certain embodiments, the 3' end of the promoter is linked to the 5' end of the polynucleotide encoding the signal peptide.

In certain embodiments, the promoter comprises a nucleotide sequence set forth in SEQ ID NO: 23.

In certain embodiments, the nucleic acid molecule capable of encoding RdCVF comprises a polyadenylation (PolyA) signal site.

In certain embodiments, the PolyA signal site is located at 3' end of the polynucleotide encoding RdCVF and operably linked to the polynucleotide encoding RdCVF.

In certain embodiments, the 3' end of the polynucleotide encoding RdCVF is linked to the 5' end of the PolyA signal site.

In certain embodiments, the PolyA signal site comprises a nucleotide sequence set forth in SEQ ID NO: 25.

In certain embodiments, the nucleic acid molecule capable of encoding RdCVF sequentially comprises, in 5' to 3' direction: the promoter, the polynucleotide encoding the signal peptide, the polynucleotide encoding RdCVF, and the PolyA signal site.

In certain embodiments, the nucleic acid molecule capable of encoding RdCVF comprises a second polynucleotide encoding a second protein, wherein the second protein is different from RdCVF.

In certain embodiments, the second polynucleotide and the polynucleotide encoding RdCVF are located in the same coding sequence region.

In certain embodiments, the nucleic acid molecule capable of encoding RdCVF comprises a polynucleotide encoding a self-cleaving peptide.

In certain embodiments, the polynucleotide encoding the self-cleaving peptide is located between the second polynucleotide and the polynucleotide encoding RdCVF.

In certain embodiments, the self-cleaving peptide comprises P2A.

In certain embodiments, the polynucleotide encoding the self-cleaving peptide comprises a nucleotide sequence set forth in SEQ ID NO: 26.

In certain embodiments, the nucleic acid molecule capable of encoding RdCVF sequentially comprises, in 5' to 3' direction: the promoter, the second polynucleotide, the polynucleotide encoding the self-cleaving peptide, the polynucleotide encoding the signal peptide, the polynucleotide encoding RdCVF, and the PolyA signal site.

In certain embodiments, the nucleic acid molecule capable of encoding RdCVF sequentially comprises, in 5' to 3' direction: the promoter, the polynucleotide encoding RdCVF, the polynucleotide encoding the self-cleaving peptide, the polynucleotide encoding the signal peptide, the second polynucleotide, and the PolyA signal site.

In certain embodiments, the second polynucleotide and the polynucleotide encoding RdCVF are located in different coding sequence regions.

In certain embodiments, the nucleic acid molecule capable of encoding RdCVF comprises at least two promoters.

In certain embodiments, the nucleic acid molecule capable of encoding RdCVF comprises at least two PolyA signal sites.

In certain embodiments, the nucleic acid molecule capable of encoding RdCVF sequentially comprises, in 5' to 3' direction: the promoter, the second polynucleotide, the PolyA signal site, the promoter, the polynucleotide encoding the signal peptide, the polynucleotide encoding RdCVF, and the PolyA signal site.

In certain embodiments, the nucleic acid molecule capable of encoding RdCVF sequentially comprises, in 5' to 3' direction: the promoter, the polynucleotide encoding RdCVF, the PolyA signal site, the promoter, the polynucleotide encoding the signal peptide, the second polynucleotide, and the PolyA signal site.

In certain embodiments, the second protein comprises CYP4V2.

In certain embodiments, the CYP4V2 comprises an amino acid sequence set forth in SEQ ID NO: 35.

In certain embodiments, the second polynucleotide comprises a polynucleotide encoding CYP4V2.

In certain embodiments, the nucleic acid molecule capable of encoding RdCVF comprises a nucleotide sequence set forth in SEQ ID NO: 24.

Other aspects and advantages of the present application can be readily appreciated by those skilled in the art from the detailed descriptions below. Only exemplary embodiments of the present application are shown and described in the detailed descriptions below. As recognized by those skilled in the art, the contents of the present application enable those skilled in the art to make changes to the specific embodiments without departing from the spirit and scope of the invention disclosed in the present application. Accordingly, the accompanying drawings and the descriptions in the specification of the present application are only exemplary and not restrictive.

### DESCRIPTION OF THE DRAWINGS

The specific features of the invention disclosed in the present application are set forth in the appended claims. The characteristics and advantages of the invention disclosed in the present application can be better understood by reference to the exemplary embodiments described in detail below and the accompanying drawings. Brief descriptions of the accompanying drawings are as follows:
Fig. 1 shows the enhanced secretion of RdCVF by the cells comprising the isolated nucleic acid molecule described herein.
Fig. 2 shows the enhanced secretion of RdCVF by the cells comprising the isolated nucleic acid molecule described herein.
Figs. 3A to 3B show the enhanced secretion of RdCVF by the cells comprising the isolated nucleic acid molecule described herein.
Fig. 4 shows that the expressions of the proteins encoded by different CDSs in the isolated nucleic acid molecule described herein do not affect each other.
Fig. 5 shows that the expressions of the proteins encoded by the same CDS in the isolated nucleic acid molecule described herein do not affect each other.
Fig. 6 shows that the isolated nucleic acid molecule described herein enhances the retinal function in a mouse model.
Fig. 7 shows that the isolated nucleic acid molecule described herein enhances the maintenance function of retinal photoreceptor cells in a mouse model.

### DESCRIPTION OF EMBODIMENTS

The embodiments of the invention in the present application are described below by certain specific examples, and those skilled in the art can easily understand other advantages and effects of the invention in the present application from the contents disclosed in this specification.

### Definitions of terms

The present application is further described below: in the present application, unless otherwise specified, the scientific and technical terms used herein have the meanings commonly understood by those skilled in the art. Moreover, the related terms and laboratory procedures in protein and nucleic acid chemistry, molecular biology, cell and tissue culture, microbiology, and immunology as used herein are the terms and routine procedures widely used in the corresponding fields. Meanwhile, for a better understanding of the present application, the definitions and explanations of related terms are provided below.

As used herein, the term "isolated" generally refers to being obtained from the natural state by an artificial means. If an "isolated" substance or ingredient occurs in nature, it may be due to a change in its natural environment, or the separation of this substance from the natural environment, or both. For example, a certain non-isolated polynucleotide or polypeptide naturally exists in a living animal body, and the same polynucleotide or polypeptide with high purity isolated from this natural state is called "isolated." The term "isolated" neither excludes the admixture of artificial or synthetic substances, nor excludes the presence of other impurities that do not affect the activity of the substance.

As used herein, the term "isolated nucleic acid molecule" generally refers to an isolated form of nucleotides, deoxyribonucleotides, or ribonucleotides of any length, or analogs isolated from their natural environments or artificially synthesized.

As used herein, the term "RdCVF", also known as Rod derived cone survival factor, generally refers to a truncated thioredoxin-like protein that lacks enzymatic activity for thiol redox. RdCVF is a splicing variant of the nucleoredoxin-like 1 (Nxnl1) gene. Another splicing product of this gene is RdCVFL, an active thioredoxin that protects its binding ligand (i.e., a microtubule-associated protein TAU) from oxidation and aggregation (Elachouri *et al.,* 2015; and Fridlich *et al.,* 2009). Mice deficient in the Nxnl1 gene can exhibit age-dependent losses of rod and cone functions and cone degeneration, as well as rod and cone hypersensitivity to oxidative stress (Cronin *et al*., 2010). The expression of Nxnl1 can be rod-dependent, which is significantly reduced after rod death in retinitis pigmentosa (RP) (Delyfer *et al*., 2011; Reichman *et al*., 2010). RdCVF can protect the cone function in several different genotypes of retinitis pigmentosa (RP) models (Byrne *et al*., 2015; Le' veillard *et al*., 2004; Yang *et al*., 2009). The Nxnl1 gene is conserved among human, chimpanzee, rhesus monkey, dog, cow, rat, mouse, chicken, zebrafish, and frog. Also known as Txnl6 in human, Nxnl1 is located at 19p13.11 and comprises two exons. Nxnl1 can be ubiquitously expressed in human tissues, including lens, retina, stomach, kidney, heart, colon, and spleen, with relatively higher expression levels in lens and retina.

As used herein, the term "signal peptide" generally refers to an amino acid sequence after the initiation codon that contains hydrophobic amino acids. The signal peptide can direct a protein into subcellular organelles containing different membrane structures. In certain cases, the signal peptide can have from about 15 to about 40 amino acids in length. The N-terminus of the signal peptide can contain basic amino acids and/or hydrophobic amino acids. The signal peptide can contain a negatively charged C-terminus, which may serve as a cleavage site for the signal peptide. When a synthesized protein enters into the cavity of the endoplasmic reticulum, the signal peptide can be cleaved by signal peptidase.

As used herein, the term "operably linked" generally refers to placing the regulatory sequence necessary for the expression of a coding sequence at an appropriate position relative to the coding sequence so as to effect the expression of the coding sequence. In certain embodiments, the arrangement of coding sequences and transcription control elements in an expression vector can be represented. The control element may include promoter, enhancer, and termination element. For example, if a promoter influences the transcription or expression of a coding sequence, the promoter is operably linked to the coding sequence. In certain embodiments, "operably linked" can also refer to the ligation of a target gene into a vector such that transcription and translation control sequences within the vector exert their intended functions of regulating the transcription and translation of the target gene.

As used herein, the term "polyadenylation (PolyA) signal site" generally refers to a base sequence located at the 3' end of messenger RNA (mRNA) that can be recognized by the polyadenylation-related cleavage factor. Usually, it is also a cis-regulatory signal on the mRNA. In general, the process of tailing (i.e., polyadenylation) begins after the termination of transcription, and tens to hundreds of single adenosines are added following 3' UTR in mRNA by the polyadenylation-related cleavage factor under the regulation of the PolyA signal site. The common tailing signals include SV40, BGH, HSV, TK signals, and the like. The polyadenylation-related cleavage factors may include cleavage/polyadenylation specific factor (CPSF), cleavage stimulation factor (CstF), cleavage factor I (CFI), cleavage factor II (CFII).

As used herein, the term "self-cleaving peptide" generally refers to a peptide fragment that can induce the self-cleavage of a recombinant protein containing this peptide in the cell. For example, the self-cleaving peptide may be a 2A self-cleaving peptide. The 2A peptide is generally derived from the 2A region of the viral genome. In genetic engineering operations, the 2A peptide can divide a peptide chain translated from an open reading frame (ORF) into several independent peptide chains. In certain embodiments, if two proteins need to be expressed separately (for example, one protein needs to enter the nucleus and the other protein needs to be expressed in the cytoplasm), and it is also desired to construct only one open reading frame in the vector, then it can be achieved by inserting a segment of 2A peptide sequence into their coding regions. In certain embodiments, if a fusion protein from the fusion of two proteins has no function, a sequence encoding a 2A peptide can be inserted between the coding regions for these two proteins, or the linking peptide can be replaced with a 2A peptide, so that after the translation is completed, these two proteins are separated from each other, and folded independently, thus providing the possibility to restore the functions of these two proteins. The 2A peptide may include P2A, E2A, F2A, and T2A, all named by the virus of origin. Among them, P2A is derived from the 2A peptide of Porcine teschovirus.

As used herein, the term "CYP4V2" generally refers to a protein that is member 2 of subfamily V of cytochrome P450 family 4. Cytochrome P450, also known as CYP450, usually refers to a family of ferroheme proteins, belonging to a class of monooxygenases, and involved in the metabolism of endogenous substances or exogenous substances including drugs and environmental compounds. According to the homology degree of amino acid sequence, the members are divided into three levels: family, subfamily, and individual enzymes. The cytochrome P450 enzyme system may be abbreviated as CYP, wherein the family is represented by Arabic number, the subfamily is represented by English capital letter, and the individual enzyme is represented by Arabic number, such as CYP4V2 herein. The human CYP4V2 gene (HGNC: 23198) has a full length of 19.28kb, located at 4q35, and has 11 exons. CYP4V2 is expressed almost in all tissues, but is expressed at a higher level in the retina and retinal pigment epithelium while at a slightly lower level in the cornea tissues, and the mutations in the CYP4V2 gene may result in BCD (Li et al., Am J Hum Genet. 74:817-826, 2004).

As used herein, the term "promoter" generally refers to a deoxyribonucleic acid (DNA) sequence that enables the transcription of a particular gene. The promoter can be recognized by RNA polymerase, and initiate the transcription and synthesis of RNA. During the synthesis of ribonucleic acid (RNA), the promoter can interact with the transcription factor for regulating the gene transcription, to control the initiation time and expression degree of the gene expression (transcription). The promoter comprises the core promoter region and the regulatory region, and is located in the regulatory sequence that controls the gene expression and upstream of the gene transcription initiation site (5' direction of the DNA antisense strand), and itself has no compilation function. According to action mode and function, the promoter is divided into three categories: constitutive promoter (consistent activity in most or all tissues), specific promoter (tissue specific or specific for developmental stage), and inducible promoter (regulated by external chemical or physical signal).

As used herein, the term "vector" generally refers to a nucleic acid delivery vehicle into which a polynucleotide encoding a protein can be inserted to express the protein. Through the transformation, transduction, or transfection of a host cell by the vector, the genetic elements carried by the vector are expressed in the host cell. For example, the vector comprises: plasmid; phagemid; cosmid; artificial chromosome, such as yeast artificial chromosome (YAC), bacterial artificial chromosome (BAC), or P1-derived artificial chromosome (PAC); phage, such as λ phage or M13 phage; viral vector; and the like. A vector may contain a variety of elements controlling expressions, including promoter sequence, transcription initiation sequence, enhancer sequence, selection element, and reporter gene. Additionally, the vector may also contain a replication origin. The vector may also comprise a component contributing to the entry into a cell, such as viral particle, liposome, or protein coat, but not limited to these substances.

As used herein, the term "viral vector" generally refers to a non-wild-type recombinant viral particle serving as a gene delivery vehicle and containing a recombinant viral genome packaged inside a viral capsid. The animal virus species used as the vector may include retrovirus (including lentivirus), adenovirus, adeno-associated virus (AAV), herpesvirus (such as herpes simplex virus), poxvirus, baculovirus, papillomavirus, and papovavirus (such as SV40).

As used herein, the term "AAV vector," also known as adeno-associated viral vector, generally refers to adenovirus itself or derivatives thereof. The adeno-associated virus (AAV) generally refers to a class of single-stranded DNA viruses belonging to the Dependovirus genus in the Parvoviridae family. The AAV genome can comprise inverted terminal repeats (ITRs) at both ends of the DNA strand, and two open reading frames (ORFs). The open reading frames may include rep and cap. Rep consists of multiple overlapping genes encoding Rep proteins required for the AAV life cycle, and cap comprises overlapping nucleotide sequences encoding capsid proteins, wherein the nucleotide sequences may include VP1, VP2, and VP3. The capsid proteins interact to form the capsid. In the absence of a helper virus, AAV can integrate its genome into a specific locus (AAVS locus) on the human chromosome 19, until a helper virus rescues it from latency (Kotin *et al*., 1990). It is generally believed that AAV is predominantly in the non-integration form. The site-specific integration capability, natural deficiency, and low immunogenicity of AAV make it an ideal gene therapy vector. AAV has a variety of common serotypes, and more than 100 virus variants.

As used herein, the term "cell" can generally be or has been a single cell, cell line or cell culture of a recipient for the nucleic acid molecule or vector. The cell may comprise the nucleic acid molecule described herein or the vector described herein. The cell may include the progeny of a single cell. Due to the natural, accidental, or intentional mutation, the progeny may not necessarily be completely identical to the original parent cell (either morphologically in total DNA complement, or genomically). The cell may include a cell transfected *in vitro* with the vector described herein. The cell may be bacterial cell (e.g., *E. coli*), yeast cell, or other eukaryotic cells, such as COS cell, Chinese hamster ovary (CHO) cell, HeLa cell, HEK293 cell, COS-1 cell, NS0 cell or myeloma cell. In certain embodiments, the cell is a mammalian cell. In certain embodiments, the mammalian cell is HEK293T cell.

As used herein, the term "pharmaceutical composition" generally refers to a composition suitable for administration to a patient such as human patient. For example, the pharmaceutical composition described herein may comprise the nucleic acid molecule described herein, the vector described herein, and/or the cell described herein, and optionally a pharmaceutically acceptable adjuvant. In addition, the pharmaceutical composition may also comprise one or more (pharmaceutically effective) vehicles, stabilizers, excipients, diluents, solubilizers, surfactants, emulsifiers, and/or preservatives for suitable formulations. The acceptable ingredients of the composition are not toxic to recipients at the dosages and concentrations employed. The pharmaceutical composition of the present application includes, but is not limited to, liquid, frozen, and lyophilized compositions.

As used herein, the term "preventing" generally refers to the prophylactic administration of a combination to a healthy subject to prevent the occurrence of a certain disease or disorder. It may also include the prophylactic administration of the combination to a patient in the early stage of an allergic disease to be treated. The term "preventing" does not require 100% elimination of the likelihood of a disease or disorder; in other words, the term "preventing" generally means that the likelihood of a disease or disorder is reduced in the presence of the combination administrated.

As used herein, the term "alleviating" refers to reducing, diminishing, or retarding a certain condition, disease, disorder, or phenotype. The condition, disease, disorder, or phenotype may include subjective perceptions of the subject such as pain, dizziness, or other physiological disturbances, or focus conditions detected by medical laboratory means.

As used herein, the term "treating" generally refers to a clinical intervention for altering the natural course of the treated individual or cell in a clinical pathological process. It may include improving the disease status, eliminating lesions, or improving the prognosis.

As used herein, the term "retinal pigment epithelium (RPE)" generally refers to a layer of pigment cells immediately outside the retinal sensory nerves. The retinal pigment epithelium consists of a single layer of hexagonal cells that contain dense pigment granules. The retinal pigment epithelium (RPE) is closely connected with the underlying choroid and the upper retinal nerve cells. Its main functions may include: controlling the fluids and nutritions in the subretinal space; functioning as a blood-retinal barrier; synthesis of the growth factor for adjusting the local structure; absorption of lights and regulation of the electrical balance; regeneration and synthesis of visual pigments; phagocytosis and digestion of photoreceptor outer segments; maintenance of retinal attachment; and regeneration and repair after injury. RPE is generally considered to be an important tissue for maintaining the photoreceptor function, and is also affected by many lesions in the choroid and retina.

As used herein, the term "retinal pigment epithelium (RPE) atrophy" generally refers to degenerative changes in the retinal pigment epithelium (RPE) manifested by cell death or dysfunction. The age-related macular degeneration or retinitis pigmentosa (RP) is often accompanied by the retinal pigment epithelium atrophy. The retinitis pigmentosa (abbreviated as RP), also known as the retinal pigment lesion, usually refers to a class of inherited ocular diseases. There are three modes of inheritance: autosomal recessive, dominant, and X-linked, and dihybrid inheritance and mitochondrial inheritance are also present. The common symptoms in the early stage may be night blindness, narrowing of visual field, the ability to see the scene right ahead but the inability to see the visual field slightly to the left and right, and then the gradual disappearance of vision. RP may include uniocular primary retinitis pigmentosa, sector primary retinitis pigmentosa, central or paracentral primary retinitis pigmentosa, retinitis pigmentosa sine pigmento, albescent punctate degeneration of retina, Bietti's crystalline dystrophy, pigmented paravenous retinitis pigmentosa, preserved para-arteriolar retinal pigment epithelium retinitis pigmentosa, Leber congenital amaurosis, and retinitis pigmentosa in other syndromes.

As used herein, the term "Bietti's crystalline dystrophy (BCD)" generally refers to a class of autosomal recessive ocular diseases first described in 1937 by an Italian ophthalmologist, Dr. GB Bietti. The symptoms mainly include crystals (transparent coverings) in the cornea; small, yellow or white, crystalline deposits deposited in the photosensitive tissues of the retina; and progressive atrophy of the retina, choriocapillary, and choroid. The Bietti's crystalline dystrophy may include a disease caused by CYP4V2 gene mutation.

As used herein, the term "comprise" or "include" generally means the inclusion of expressly specified features, but without the exclusion of other elements.

As used herein, the term "about" generally refers to variations above or below the specified value within the range of 0.5%-10%, such as variations above or below the specified value within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%.

### Detailed descriptions

### RdCVF and polynucleotide encoding RdCVF

In the present application, the RdCVF may be human RdCVF. For example, the accession number of the human RdCVF protein in NCBI may be NP_612463.1.

In the present application, the RdCVF may be mouse RdCVF, rat RdCVF, or may be monkey RdCVF. For example, the accession number of the mouse RdCVF protein in UniProtKB may be Q8VC33.1.

In the present application, the RdCVF may be an intact RdCVF protein, or may comprise a functional fragment of the RdCVF protein. The functional fragment of the RdCVF protein may include the variants, truncations, and/or fragments of the RdCVF protein, as long as the functional fragment of the RdCVF protein retains the biological functions of the RdCVF protein (such as protecting cone cells; or promoting the uptake of glucose by photoreceptor cells).

In the present application, the RdCVF may comprise an amino acid sequence set forth in SEQ ID NO: 34.

In the present application, the polynucleotide encoding RdCVF may encode and express the RdCVF protein. For example, the polynucleotide encoding RdCVF may comprise a codon-optimized nucleotide sequence.

In the present application, the codon optimization generally refers to a method of increasing the expression level of the encoded protein by preferred codons. For example, the codon optimization may include replacing the synonymous codons with highest frequencies in the host genome with the codons of a donor. For example, the codon optimization may include utilizing the most abundant codons in the host to encode the amino acids in the optimized sequence. For example, the means for codon optimization may include adjusting the CG content in the nucleotide sequence, removing the motifs unfavorable for expression, and/or simplifying the secondary structure of post-transcriptional mRNA. In the present application, the codon optimization may be implemented by the means well-known to those skilled in the art, for example, the OPTGENE^{™} program may be used to select a suitable codon-optimized nucleotide sequence.

In the present application, the polynucleotide encoding RdCVF may comprise a nucleotide sequence set forth in SEQ ID NO: 22.

### Signal peptide and polynucleotide encoding signal peptide

In the present application, the type of the signal peptide may include Sec/SPI (i.e., Sec signal peptide), Sec/SPII (i.e., lipoprotein signal peptide), and/or Tat/SPI (i.e., Tat signal peptide). The signal peptide may be cleaved by a signal peptidase after directing the transport of the protein. The signal peptidase may include type I signal peptidase (SPaseI, i.e., SPI), type II signal peptidase (SPaseII, i.e., SPII), and type III signal peptidase (SPaseIII). For example, the signal peptide may be removed by type I signal peptidase (SPaseI).

In the present application, the signal peptide may comprise three regions, that is, 1) N-terminus comprising a basic amino terminus with positively charged amino acids; 2) hydrophobic intermediate sequence comprising neutral amino acids (which may also be considered as the main functional region of the signal peptide); and 3) C-terminus comprising negatively charged amino acids, which may comprise the cleavage site of the signal peptide.

In the present application, the signal peptide has at least 9 amino acids in length. For example, the signal peptide may have at least 10 amino acids, at least 11 amino acids, at least 12 amino acids, at least 13 amino acids, at least 14 amino acids, at least 15 amino acids or more in length. For example, the signal peptide may have about 9 to about 70 amino acids, for example, about 10 to about 70 amino acids, about 15 to about 70 amino acids, or about 15 to about 40 amino acids in length.

In the present application, the signal peptide may be selected from the group consisting of GL, RS1, NDP, GAS6, GUCY2D, and CRB2.

In the present application, the signal peptide may comprise an amino acid sequence set forth in any one of SEQ ID NOs: 27-31 and 33.

In the present application, the polynucleotide encoding the signal peptide may comprise a nucleotide sequence set forth in any one of SEQ ID NOs: 15-19 and 21.

### Fusion protein

The present application provides a fusion protein comprising RdCVF and a signal peptide, wherein the signal peptide is an amino acid sequence set forth in any one of SEQ ID NOs: 27-31 and 33.

Compared with RdCVF, the expression level of the fusion protein described herein is significantly increased in the cell. And/or, compared with RdCVF, the secretion amount of the fusion protein described herein is significantly increased in the cell.

In the present application, the RdCVF in the fusion protein may be located at the C-terminus of the signal peptide. For example, the N-terminus of the RdCVF may be directly or indirectly linked to the C-terminus of the signal peptide.

For example, the fusion protein may sequentially comprise, from N-terminus to C-terminus, the signal peptide and the RdCVF. For example, the fusion protein may comprise an amino acid sequence set forth in any one of SEQ ID NOs: 48-54.

In the present application, the fusion protein may further comprise a second protein, wherein the second protein is a protein different from the RdCVF. In the present application, the second protein may comprise a protein expressed in the retina or a functional fragment thereof. In the present application, the second protein may comprise a protein or a functional fragment thereof that is effective in treating, alleviating, and/or preventing a disease or disorder associated with retinal pigment epithelium (RPE) atrophy. For example, the second protein may comprise a protein or a functional fragment thereof that is effective in treating, alleviating, and/or preventing Bietti's crystalline dystrophy.

For example, the second protein may comprise CYP4V2. For example, the CYP4V2 may be human CYP4V2. For example, the human CYP4V2 may comprise an amino acid sequence set forth in SEQ ID NO: 34.

In the present application, the C-terminus of the second protein may be directly or indirectly linked to the N-terminus of the signal peptide.

In the present application, the fusion protein may sequentially comprise, from N-terminus to C-terminus, the second protein, the signal peptide, and the RdCVF. For example, the fusion protein may comprise an amino acid sequence set forth in SEQ ID NO: 55.

In the present application, the fusion protein may be an intermediate product in the translation process, and after the post-translational modification, the signal peptide in the fusion protein may be removed (for example, it may be cleaved by a signal peptidase).

### Isolated nucleic acid molecule

The present application provides an isolated nucleic acid molecule comprising a) the polynucleotide encoding RdCVF described herein and b) the polynucleotide encoding the signal peptide described herein.

In the present application, the polynucleotide encoding the signal peptide may be located at 5' end of the polynucleotide encoding RdCVF and operably linked to the polynucleotide encoding RdCVF. For example, the 3' end of the polynucleotide encoding the signal peptide may be linked to the 5' end of the polynucleotide encoding RdCVF.

In the present application, the polynucleotide encoding the signal peptide may be located at 3' end of the polynucleotide encoding RdCVF and operably linked to the polynucleotide encoding RdCVF. For example, the 5' end of the polynucleotide encoding the signal peptide may be linked to the 3' end of the polynucleotide encoding RdCVF.

In the present application, the isolated nucleic acid molecule may comprise a promoter.

In the present application, the promoter may be located at 5' end of the polynucleotide encoding RdCVF and operably linked to the polynucleotide encoding RdCVF.

In the present application, the promoter may be located at 5' end of the polynucleotide encoding the signal peptide and operably linked to the polynucleotide encoding the signal peptide. For example, the 3' end of the promoter may be linked to the 5' end of the polynucleotide encoding the signal peptide.

In the present application, the promoter may comprise a nucleotide sequence set forth in SEQ ID NO: 23. For example, the promoter may include CAG, which may comprise a nucleotide sequence set forth in SEQ ID NO: 23.

In the present application, the isolated nucleic acid molecule may comprise a polyadenylation (PolyA) signal site.

In the present application, the PolyA signal site may be located at 3' end of the polynucleotide encoding RdCVF and operably linked to the polynucleotide encoding RdCVF. For example, the 3' end of the polynucleotide encoding RdCVF may be linked to the 5' end of the PolyA signal site.

In the present application, the PolyA signal site may comprise a nucleotide sequence set forth in SEQ ID NO: 25. For example, the PolyA signal site may include BGH, which may comprise a nucleotide sequence set forth in SEQ ID NO: 25.

In the present application, the isolated nucleic acid molecule may sequentially comprise, in 5' to 3' direction: the promoter, the polynucleotide encoding the signal peptide, the polynucleotide encoding RdCVF, and the PolyA signal site.

For example, the isolated nucleic acid molecule may sequentially comprise, in 5' to 3' direction: the promoter CAG, the polynucleotide encoding the signal peptide GL, the polynucleotide encoding RdCVF, and the PolyA signal site BGH. For example, the isolated nucleic acid molecule may comprise a nucleotide sequence set forth in SEQ ID NO: 37.

For example, the isolated nucleic acid molecule may sequentially comprise, in 5' to 3' direction: the promoter CAG, the polynucleotide encoding the signal peptide GUCY2D, the polynucleotide encoding RdCVF, and the PolyA signal site BGH. For example, the isolated nucleic acid molecule may comprise a nucleotide sequence set forth in SEQ ID NO: 38.

For example, the isolated nucleic acid molecule may sequentially comprise, in 5' to 3' direction: the promoter CAG, the polynucleotide encoding the signal peptide RS1, the polynucleotide encoding RdCVF, and the PolyA signal site BGH. For example, the isolated nucleic acid molecule may comprise a nucleotide sequence set forth in SEQ ID NO: 39.

For example, the isolated nucleic acid molecule may sequentially comprise, in 5' to 3' direction: the promoter CAG, the polynucleotide encoding the signal peptide NDP, the polynucleotide encoding RdCVF, and the PolyA signal site BGH. For example, the isolated nucleic acid molecule may comprise a nucleotide sequence set forth in SEQ ID NO: 40.

For example, the isolated nucleic acid molecule may sequentially comprise, in 5' to 3' direction: the promoter CAG, the polynucleotide encoding the signal peptide GAS6, the polynucleotide encoding RdCVF, and the PolyA signal site BGH. For example, the isolated nucleic acid molecule may comprise a nucleotide sequence set forth in SEQ ID NO: 41.

For example, the isolated nucleic acid molecule may sequentially comprise, in 5' to 3' direction: the promoter CAG, the polynucleotide encoding the signal peptide CRB2, the polynucleotide encoding RdCVF, and the PolyA signal site BGH. For example, the isolated nucleic acid molecule may comprise a nucleotide sequence set forth in SEQ ID NO: 43.

In the present application, the isolated nucleic acid molecule may further comprise a second polynucleotide encoding a second protein, wherein the second protein is different from RdCVF.

In the present application, the second polynucleotide and the polynucleotide encoding RdCVF may be located in the same coding sequence (CDS) region. In the present application, the coding region may comprise a part of the gene capable of transcribing mRNA and thereby synthesizing the corresponding protein.

In the present application, the isolated nucleic acid molecule may comprise a polynucleotide encoding a self-cleaving peptide. In the present application, the self-cleaving peptide may be a polypeptide fragment capable of producing two or more proteins from a single mRNA transcript. For example, the self-cleaving peptide may be 2A peptide. The 2A peptide may induce the self-cleavage of the recombinant protein comprising the 2A peptide. For example, the 2A peptide may be selected from the group consisting of P2A, T2A, E2A, and F2A (see Cleavage efficiency of 2A linkers in human cell lines, zebrafish and mice, PLoS One. 6:e18556 (2011)). For example, the self-cleaving peptide may include P2A.

In the present application, the polynucleotide encoding the self-cleaving peptide may comprise a nucleotide sequence set forth in SEQ ID NO: 26.

In the present application, the polynucleotide encoding the self-cleaving peptide may be located between the second polynucleotide and the polynucleotide encoding RdCVF.

In the present application, the isolated nucleic acid molecule may sequentially comprise, in 5' to 3' direction: the promoter, the second polynucleotide, the polynucleotide encoding the self-cleaving peptide, the polynucleotide encoding the signal peptide, the polynucleotide encoding RdCVF, and the PolyA signal site.

For example, the isolated nucleic acid molecule may sequentially comprise, in 5' to 3' direction: the promoter, the second polynucleotide encoding CYP4V2, the polynucleotide encoding the self-cleaving peptide, the polynucleotide encoding the signal peptide GL, the polynucleotide encoding RdCVF, and the PolyA signal site.

For example, the isolated nucleic acid molecule may sequentially comprise, in 5' to 3' direction: the promoter CAG, the second polynucleotide encoding CYP4V2, the polynucleotide encoding the self-cleaving peptide P2A, the polynucleotide encoding the signal peptide GL, the polynucleotide encoding RdCVF, and the PolyA signal site BGH. For example, the isolated nucleic acid molecule may comprise a nucleotide sequence set forth in SEQ ID NO: 47.

In the present application, the isolated nucleic acid molecule may sequentially comprise, in 5' to 3' direction: the promoter, the polynucleotide encoding RdCVF, the polynucleotide encoding the self-cleaving peptide, the polynucleotide encoding the signal peptide, the second polynucleotide, and the PolyA signal site.

In the present application, the second polynucleotide and the polynucleotide encoding RdCVF are located in different coding sequence regions.

In the present application, the isolated nucleic acid molecule may comprise at least two promoters. For example, the promoter may include CAG, which may comprise a nucleotide sequence set forth in SEQ ID NO: 23.

In the present application, the isolated nucleic acid molecule may comprise at least two PolyA signal sites. For example, the PolyA signal site may include BGH, which may comprise a nucleotide sequence set forth in SEQ ID NO: 25.

In the present application, the second protein may comprise CYP4V2. For example, the second polynucleotide may comprise a polynucleotide encoding CYP4V2.

In the present application, the isolated nucleic acid molecule may sequentially comprise, in 5' to 3' direction: the promoter, the second polynucleotide, the PolyA signal site, the promoter, the polynucleotide encoding the signal peptide, the polynucleotide encoding RdCVF, and the PolyA signal site.

For example, in the present application, the isolated nucleic acid molecule may sequentially comprise, in 5' to 3' direction: the promoter, the second polynucleotide, the PolyA signal site, the promoter, the polynucleotide encoding the signal peptide GL, the polynucleotide encoding RdCVF, and the PolyA signal site.

For example, in the present application, the isolated nucleic acid molecule may sequentially comprise, in 5' to 3' direction: the promoter, the second polynucleotide encoding CYP4V2, the PolyA signal site, the promoter, the polynucleotide encoding the signal peptide GL, the polynucleotide encoding RdCVF, and the PolyA signal site.

For example, in the present application, the isolated nucleic acid molecule may sequentially comprise, in 5' to 3' direction: the promoter CAG, the second polynucleotide encoding CYP4V2, the PolyA signal site BGH, the promoter CAG, the polynucleotide encoding the signal peptide GL, the polynucleotide encoding RdCVF, and the PolyA signal site BGH. For example, the isolated nucleic acid molecule may comprise a nucleotide sequence set forth in SEQ ID NO: 45.

In the present application, the isolated nucleic acid molecule may sequentially comprise, in 5' to 3' direction: the promoter, the polynucleotide encoding RdCVF, the PolyA signal site, the promoter, the polynucleotide encoding the signal peptide, the second polynucleotide, and the PolyA signal site.

In the present application, the isolated nucleic acid molecule may comprise a nucleotide sequence set forth in any one of SEQ ID NOs: 37-41, 43, 45, and 47.

### Vector, cell, pharmaceutical composition, method, and use

In another aspect, the present application provides a vector, wherein the vector may comprise the isolated nucleic acid molecule described herein.

In the present application, the vector may comprise a viral vector.

In the present application, the vector may also include plasmid, phagemid, cosmid, artificial chromosome (such as yeast artificial chromosome (YAC), bacterial artificial chromosome (BAC), or P1-derived artificial chromosome (PAC)), phage (such as λ phage or M13 phage), and viral vectors.

For example, the viral vector may include retrovirus (including lentivirus), adenovirus, adeno-associated virus (AAV vector), herpesvirus (such as herpes simplex virus), poxvirus, baculovirus, papillomavirus, and papovavirus (such as SV40) vectors.

In the present application, the vector may be a viral vector, and the viral vector may comprise an AAV vector. The AAV vector may be an adeno-associated virus vector. The AAV vector may serve as a vector required for gene therapy. For example, the adeno-associated virus vector (AAV vector) gene may comprise an inverted terminal repeat (ITR) and an open reading frame (ORF), wherein the open reading frame may include a polynucleotide encoding Rep protein, and may also include a polynucleotide encoding a capsid.

For example, the adeno-associated virus vector (AAV vector) may also include a recombinant adeno-associated virus vector (rAAV vector).

In the present application, the AAV vectors may have different serotypes. For example, the AAV vector may be AAV8.

In another aspect, the present application provides a cell, wherein the cell may comprise the isolated nucleic acid molecule described herein or the vector described herein.

In another aspect, the present application provides a cell, which may express the fusion protein described herein under the condition that the fusion protein described herein may be expressed.

In the present application, the cell may be a cell in which the nucleic acid molecule is expressed.

For example, the cell may include the progeny of a single cell. The progeny may not necessarily be completely identical to the original parent cell (either morphologically in total DNA complement, or genomically).

For example, the cell may also include a cell transfected *in vitro* with the vector described herein.

For example, the cell may include bacterial cell (e.g., *E. coli*), yeast cell, or other eukaryotic cells, such as COS cell, Chinese hamster ovary (CHO) cell, HeLa cell, HEK293 cell, COS-1 cell, NS0 cell or myeloma cell, and 293T cell.

For example, the cell may include a cell from a patient with Bietti's crystalline dystrophy. For example, the cell may include somatic or stem cell.

For example, the cell may include retinal cell, corneal cell, choroidal cell, lens cell, nerve cell, RPE cell, and stem cell, and the stem cell may include induced pluripotent stem cell (iPSC), embryonic stem cell (ESC), mesenchymal stem cell (MSC), adult stem cell, or any cell differentiated from stem cell.

For example, the retinal cell, corneal cell, choroidal cell, lens cell, nerve cell, or RPE cell can be induced and differentiated from the stem cell.

For example, the cell may include ARPE-19 cell, or human iPSC-induced RPE cell.

In another aspect, the present application provides a pharmaceutical composition, wherein the pharmaceutical composition may comprise the isolated nucleic acid molecule described herein, the vector described herein, and/or the cell described herein.

For example, the pharmaceutical composition may also optionally comprise a pharmaceutically acceptable adjuvant.

For example, the pharmaceutical composition may also comprise one or more (pharmaceutically effective) vehicles, stabilizers, excipients, diluents, solubilizers, surfactants, emulsifiers, and/or preservatives for suitable formulations.

For example, the acceptable ingredients of the composition are not toxic to recipients at the dosages and concentrations employed.

For example, the pharmaceutical composition includes, but is not limited to, liquid, frozen, and lyophilized compositions.

For example, the pharmaceutically acceptable adjuvant may include any and all solvents, dispersion media, coatings, isotonic agents, and absorption delaying agents compatible with the pharmaceutical administration, which are generally safe, non-toxic, and neither biologically nor otherwise undesirable.

For example, the pharmaceutical composition may involve parenteral, transdermal, intracavity, intraarterial, intrathecal, and/or intraocular administration, or direct injection into tissues.

For example, the pharmaceutical composition may be administrated to a patient or subject by instillation, infusion, or injection.

For example, the pharmaceutical composition may be uninterruptedly (or continuously) administrated.

For example, the uninterrupted (or continuous) administration may be achieved by a small pump system worn by a patient for measuring the influx of the therapeutic agent into the patient, as described in WO2015/036583.

In the present application, the subject may include human and non-human animals.

For example, the subject may include, but not limited to, cats, dogs, horses, pigs, cows, sheep, rabbits, mice, rats, or monkeys.

In another aspect, the present application provides the use of the isolated nucleic acid molecule described herein, the vector described herein, the cell described herein, and/or the pharmaceutical composition described herein in the manufacture of a medicament for treating, alleviating, and/or preventing a disease or disorder associated with retinal pigment epithelium (RPE) atrophy.

In another aspect, the present application provides the isolated nucleic acid molecule described herein, the vector described herein, the cell described herein, and/or the pharmaceutical composition described herein for treating, alleviating, and/or preventing a disease or disorder associated with retinal pigment epithelium (RPE) atrophy.

In another aspect, the present application provides a method of treating, alleviating, and/or preventing a disease or disorder associated with retinal pigment epithelium (RPE) atrophy, comprising a step of: administrating the isolated nucleic acid molecule described herein, the vector described herein, the cell described herein, and/or the pharmaceutical composition described herein to a subject in need thereof.

In the present application, the disease or disorder associated with retinal pigment epithelium (RPE) atrophy may include age-related macular degeneration or retinitis pigmentosa (RP).

For example, the retinitis pigmentosa may include uniocular primary retinitis pigmentosa, sector primary retinitis pigmentosa, central or paracentral primary retinitis pigmentosa, retinitis pigmentosa sine pigmento, albescent punctate degeneration of retina, Bietti's crystalline dystrophy, pigmented paravenous retinitis pigmentosa, preserved para-arteriolar retinal pigment epithelium retinitis pigmentosa, Leber congenital amaurosis, and retinitis pigmentosa in other syndromes.

For example, the retinitis pigmentosa may include Bietti's crystalline dystrophy.

In another aspect, the present application provides a method of increasing the expression level of RdCVF, wherein the method may include a step of: making a nucleic acid molecule capable of encoding RdCVF to contain a polynucleotide encoding a signal peptide.

In the present application, the nucleic acid molecule capable of encoding RdCVF may comprise the isolated nucleic acid molecule described herein.

In the present application, the method comprises an in vitro method and/or an in vivo method. For example, the method may include making a cell to contain the isolated nucleic acid molecule described herein or the vector described herein under in vitro or ex vivo conditions. Also, for example, the method may include making a cell to contain the isolated nucleic acid molecule described herein or the vector described herein under in vivo conditions (e.g., by gene editing).

In the present application, compared with the nucleic acid molecule without the polynucleotide encoding the signal peptide, the expression level of RdCVF secreted by the cell having the nucleic acid molecule comprising the polynucleotide encoding the signal peptide is significantly increased. For example, it may be increased by at least 5%, at least 10%, at least 15%, at least 20%, at least 25%, at least 50%, at least 100%, at least 200%, or more.

In the present application, the cell may comprise a mammalian cell. For example, the cell may include retinal cell, corneal cell, choroidal cell, lens cell, nerve cell, RPE cell, and stem cell, and the stem cell may include induced pluripotent stem cell (iPSC), embryonic stem cell (ESC), mesenchymal stem cell (MSC), adult stem cell, or any cell differentiated from stem cell. For example, the retinal cell, corneal cell, choroidal cell, lens cell, nerve cell, or RPE cell can be induced and differentiated from the stem cell. For example, the cell may include ARPE-19 cell, or human iPSC-induced RPE cell.

Without intention to be limited by any theory, the following Examples are only intended to illustrate the fusion protein, isolated nucleic acid molecule, and use thereof, and are not intended to limit the scope of the claimed invention.

### Examples

### Example 1 GL signal peptide enhances the secretion of RdCVF

(1) Using PEI (purchased from Polysciences, 24765-1), the plasmids pAV-CAG-RdCVF-BGH (SEQ ID NO.1) and pAV-CAG-spGL-RdCVF-BGH (SEQ ID NO.2) were respectively transfected into 293T cells (purchased from ATCC, CRL-3216) plated onto 25 mm plates in advance, with a cell density of about 70%. The medium was replaced after 6-8 hours.
(2) After 48 hours of transfection, the cell medium supernatant was transferred into 1.5 ml EP tubes, and centrifuged at room temperature at 5,000 rpm for 5 min to remove cell debris. 100 µl of supernatant after centrifugation was taken and 20 µl of 5* protein SDS loading buffer was added, then heated at 95°C for 10 min, thereby serving as the sample to be tested for extracellular secretion of RdCVF protein.
(3) The adherent cells on the remaining culture dishes were washed with PBS once and 200 µl of RIPA lysis buffer (purchased from Beijing Applygen, C1053-100) were added. The cells were scraped off and then transferred into 1.5 ml EP tubes. The cells were lysed on ice for 30 min, and centrifuged at 4°C at 12,000 rpm for 30 min. 100 µl of supernatant after centrifugation was taken and 20 µl of 5* protein SDS loading buffer was added, then heated at 95°C for 10 min, thereby serving as the sample to be tested for intracellular expression of RdCVF protein.
(4) By running in the page gel, the extracellular and intracellular expression levels and secretion conditions for RdCVF protein were detected by Western blot, respectively. The following antibodies were used: CYP4V2 antibody (purchased from Atlas, HPA029122), actin antibody (purchased from Abclonal, AC026), RdCVF antibody (i.e., TXNL6 antibody, H00115861-D01P), and goat-anti-rabbit antibody (purchased from Abclonal, AS014).

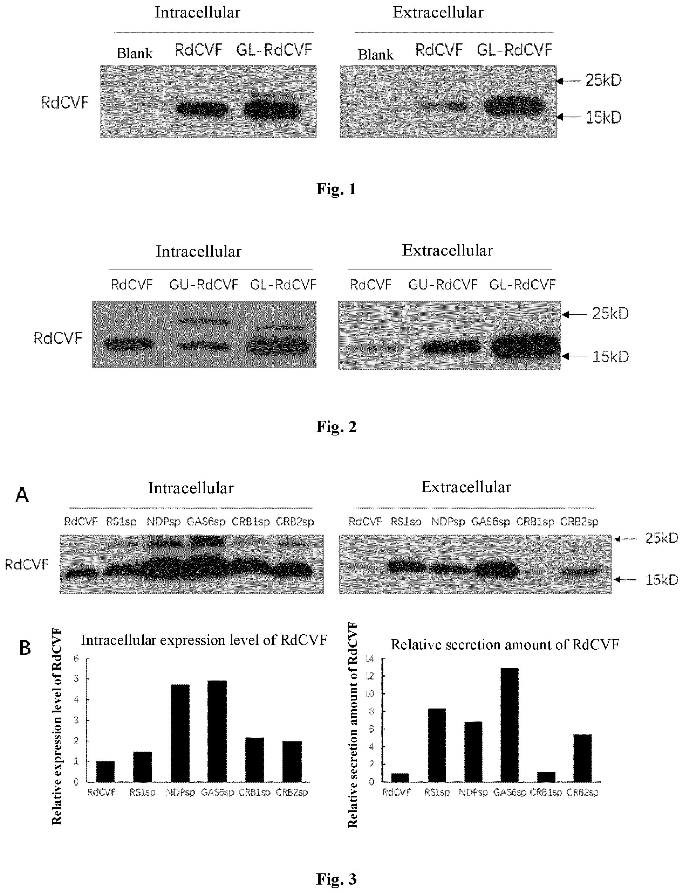

The results were shown in Fig. 1, wherein "intracellular" and "extracellular" involved the Western blot detection results of the sample to be tested for extracellular secretion of RdCVF protein and the sample to be tested for intracellular expression of RdCVF protein, respectively; and wherein the blank was a blank control, RdCVF was pAV-CAG-RdCVF-BGH transfection group, and GL-RdCVF was pAV-CAG-spGL-RdCVF-BGH transfection group. It could be known from the results in Fig. 1 that after the GL signal peptide sequence was inserted into the RdCVF coding region, the expression level of RdCVF was slightly enhanced, and the secretion function was significantly enhanced.

### Example 2 GUCY2D signal peptide enhances the secretion of RdCVF

(1) Using PEI (purchased from Polysciences, 24765-1), the plasmids pAV-CAG-RdCVF-BGH (SEQ ID NO.1), pAV-CAG-spGL-RdCVF-BGH (SEQ ID NO.2), and pAV-CAG-spGUCY2D-RdCVF-BGH (SEQ ID NO.3) were respectively transfected into 293T cells (purchased from ATCC, CRL-3216) plated onto 25 mm plates in advance, with a cell density of about 70%. The medium was replaced after 6-8 hours.
(2) After 48 hours of transfection, the cell medium supernatant was transferred into 1.5 ml EP tubes, and centrifuged at room temperature at 5,000 rpm for 5 min to remove cell debris. 100 µl of supernatant after centrifugation was taken and 20 µl of 5* protein SDS loading buffer was added, then heated at 95°C for 10 min, thereby serving as the sample to be tested for extracellular secretion of RdCVF protein.
(3) The adherent cells on the remaining culture dishes were washed with PBS once and 200 µl of RIPA lysis buffer (purchased from Beijing Applygen, C1053-100) were added. The cells were scraped off and then transferred into 1.5 ml EP tubes. The cells were lysed on ice for 30 min, and centrifuged at 4°C at 12,000 rpm for 30 min. 100 µl of supernatant after centrifugation was taken and 20 µl of 5* protein SDS loading buffer was added, then heated at 95°C for 10 min, thereby serving as the sample to be tested for intracellular expression of RdCVF protein.
(4) By running in the page gel, the extracellular and intracellular expression levels and secretion conditions for RdCVF protein were detected by Western blot, respectively.

The following antibodies were used: CYP4V2 antibody (purchased from Atlas, HPA029122), actin antibody (purchased from Abclonal, AC026), RdCVF antibody (i.e., TXNL6 antibody, H00115861-D01P), and goat-anti-rabbit antibody (purchased from Abclonal, AS014).

The results were shown in Fig. 2, wherein "intracellular" and "extracellular" involved the Western blot detection results of the sample to be tested for extracellular secretion of RdCVF protein and the sample to be tested for intracellular expression of RdCVF protein, respectively; and wherein RdCVF was pAV-CAG-RdCVF-BGH transfection group, GL-RdCVF was pAV-CAG-spGL-RdCVF-BGH transfection group, and GU-RdCVF was pAV-CAG-spGUCY2D-RdCVF-BGH transfection group. It could be known from the results in Fig. 2 that after the GUCY2D signal peptide sequence was inserted into the RdCVF coding region, the expression level of intracellular RdCVF was slightly reduced, but the secretion amount of extracellular RdCVF was significantly increased.

### Example 3 RS1, NDP, GAS6, and CRB2 signal peptides enhance the secretion of RdCVF

(1) Using PEI (purchased from Polysciences, 24765-1), the plasmids pAV-CAG-RdCVF-BGH (SEQ ID NO.1), pAV-CAG-spRS1-RdCVF-BGH (SEQ ID NO.4), pAV-CAG-spNDP-RdCVF-BGH (SEQ ID NO.5), pAV-CAG-spGAS6-RdCVF-BGH (SEQ ID NO.6), pAV-CAG-spCRB1-RdCVF-BGH (SEQ ID NO.7), and pAV-CAG-spCRB2-RdCVF-BGH (SEQ ID NO.8) were respectively transfected into 293T cells (purchased from ATCC, CRL-3216) plated onto 25 mm plates in advance, with a cell density of about 70%. The medium was replaced after 6-8 hours.
(2) After 48 hours of transfection, the cell medium supernatant was transferred into 1.5 ml EP tubes, and centrifuged at room temperature at 5,000 rpm for 5 min to remove cell debris. 100 µl of supernatant after centrifugation was taken and 20 µl of 5* protein SDS loading buffer was added, then heated at 95°C for 10 min, thereby serving as the sample to be tested for extracellular secretion of RdCVF protein.
(3) The adherent cells on the remaining culture dishes were washed with PBS once and 200 µl of RIPA lysis buffer (purchased from Beijing Applygen, C1053-100) were added. The cells were scraped off and then transferred into 1.5 ml EP tubes. The cells were lysed on ice for 30 min, and centrifuged at 4°C at 12,000 rpm for 30 min. 100 µl of supernatant after centrifugation was taken and 20 µl of 5* protein SDS loading buffer was added, then heated at 95°C for 10 min, thereby serving as the sample to be tested for intracellular expression of RdCVF protein.
(4) By running in the page gel, the extracellular and intracellular expression levels and secretion conditions for RdCVF protein were detected by Western blot, respectively.

The following antibodies were used: CYP4V2 antibody (purchased from Atlas, HPA029122), actin antibody (purchased from Abclonal, AC026), RdCVF antibody (i.e., TXNL6 antibody, H00115861-D01P), and goat-anti-rabbit antibody (purchased from Abclonal, AS014).

The results were shown in Figs. 3A-3B, wherein "intracellular" and "extracellular" involved the detection results of the sample to be tested for extracellular secretion of RdCVF protein and the sample to be tested for intracellular expression of RdCVF protein, respectively. Fig. 3A showed the Western blot detection results of RdCVF protein expression; Fig. 3B showed the quantitative results of the intracellular expression level and the extracellular relative secretion amount for RdCVF protein. RdCVF was pAV-CAG-RdCVF-BGH transfection group, RS1sp was pAV-CAG-spRS1-RdCVF-BGH transfection group, NDPsp was pAV-CAG-spNDP-RdCVF-BGH transfection group, GAS6sp was pAV-CAG-spGAS6-RdCVF-BGH transfection group, CRB 1 sp was pA V -CAG-spCRB 1-RdCVF -BGH transfection group, and CRB2sp was pAV-CAG-spCRB2-RdCVF-BGH transfection group.

It could be known from the results in Fig. 3 that after the RS1, NDP, GAS6, CRB1, and CRB2 signal peptide sequences were inserted into the RdCVF coding region, the expression level of intracellular RdCVF was unchanged or slightly increased. Under extracellular conditions, after the RS1, NDP, GAS6, and CRB2 signal peptide sequences were inserted into the RdCVF coding region, the secretion amount of RdCVF was significantly increased; however, after the CRB1 signal peptide sequence was inserted into the RdCVF coding region, the secretion amount of RdCVF was not significantly changed.

### Example 4

(1) Using PEI (purchased from Polysciences, 24765-1), the plasmids pAV-CAG-CYP4V2-BGH-CAG-RdCVF-BGH (SEQ ID NO.9) and pAV-CAG-CYP4V2-BGH-CAG-spGL-RdCVF-BGH (SEQ ID NO. 10) were transfected into 293T cells (purchased from ATCC, CRL-3216) plated onto 25 mm plates in advance, with a cell density of about 70%. The medium was replaced after 6-8 hours.
(2) After 48 hours of transfection, the cell medium supernatant was transferred into 1.5 ml EP tubes, and centrifuged at room temperature at 5,000 rpm for 5 min to remove cell debris. 100 µl of supernatant after centrifugation was taken and 20 µl of 5* protein SDS loading buffer was added, then heated at 95°C for 10 min, thereby serving as the sample to be tested for extracellular secretion of RdCVF protein.
(3) The adherent cells on the remaining culture dishes were washed with PBS once and 200 µl of RIPA lysis buffer (purchased from Beijing Applygen, C1053-100) were added. The cells were scraped off and then transferred into 1.5 ml EP tubes. The cells were lysed on ice for 30 min, and centrifuged at 4°C at 12,000 rpm for 30 min. 100 µl of supernatant after centrifugation was taken and 20 µl of 5* protein SDS loading buffer was added, then heated at 95°C for 10 min, thereby serving as the sample to be tested for intracellular expression of RdCVF protein.
(4) By running in the page gel, the extracellular and intracellular expression levels and secretion conditions for RdCVF protein were detected by Western blot, respectively. The following antibodies were used: CYP4V2 antibody (purchased from Atlas, HPA029122), actin antibody (purchased from Abclonal, AC026), RdCVF antibody (i.e., TXNL6 antibody, H00115861-D01P), and goat-anti-rabbit antibody (purchased from Abclonal, AS014).

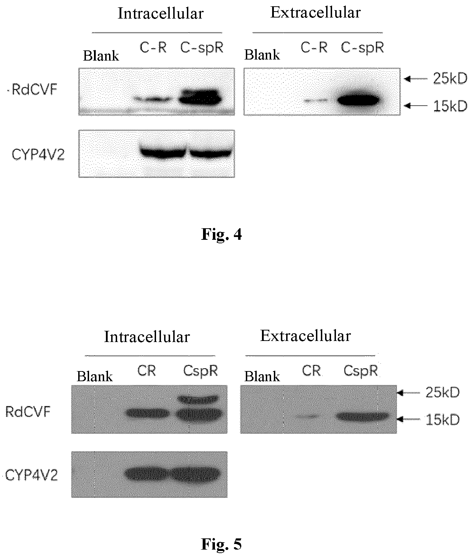

The results were shown in Fig. 4, wherein "intracellular" and "extracellular" involved the Western blot detection results of the sample to be tested for extracellular secretion of RdCVF protein and the sample to be tested for intracellular expression of RdCVF protein, respectively; and wherein the blank was a blank control, C-R was pAV-CAG-CYP4V2-BGH-CAG-RdCVF-BGH transfection group, and C-spR was pAV-CAG-CYP4V2-BGH-CAG-spGL-RdCVF-BGH transfection group. It could be known from the results in Fig. 4 that after the signal peptide sequence was inserted into the RdCVF coding region, the expression of CYP4V2 was not affected, while the intracellular expression level of RdCVF was slightly enhanced and the extracellular secretion amount was significantly increased. It could be known that the signal peptide at the N-terminus of RdCVF could enhance the secretion of RdCVF without affecting the expression of different CDS on the same vector.

### Example 5

(1) Using PEI (purchased from Polysciences, 24765-1), the plasmids pAV-CAG-CYP4V2-P2A-RdCVF-BGH (SEQ ID NO.11) and pAV-CAG-CYP4V2-P2A-spGL-RdCVF-BGH (SEQ ID NO.12) were transfected into 293T cells (purchased from ATCC, CRL-3216) plated onto 25 mm plates in advance, with a cell density of about 70%. The medium was replaced after 6-8 hours.
(2) After 48 hours of transfection, the cell medium supernatant was transferred into 1.5 ml EP tubes, and centrifuged at room temperature at 5,000 rpm for 5 min to remove cell debris. 100 µl of supernatant after centrifugation was taken and 20 µl of 5* protein SDS loading buffer was added, then heated at 95°C for 10 min, thereby serving as the sample to be tested for extracellular secretion of RdCVF protein.
(3) The adherent cells on the remaining culture dishes were washed with PBS once and 200 µl of RIPA lysis buffer (purchased from Beijing Applygen, C1053-100) were added. The cells were scraped off and then transferred into 1.5 ml EP tubes. The cells were lysed on ice for 30 min, and centrifuged at 4°C at 12,000 rpm for 30 min. 100 µl of supernatant after centrifugation was taken and 20 µl of 5* protein SDS loading buffer was added, then heated at 95°C for 10 min, thereby serving as the sample to be tested for intracellular expression of RdCVF protein.
(4) By running in the page gel, the extracellular and intracellular expression levels and secretion conditions for RdCVF protein were detected by Western blot, respectively. The following antibodies were used: CYP4V2 antibody (purchased from Atlas, HPA029122), actin antibody (purchased from Abclonal, AC026), RdCVF antibody (i.e., TXNL6 antibody, H00115861-D01P), and goat-anti-rabbit antibody (purchased from Abclonal, AS014).

The results were shown in Fig. 5, wherein "intracellular" and "extracellular" involved the Western blot detection results of the sample to be tested for extracellular secretion of RdCVF protein and the sample to be tested for intracellular expression of RdCVF protein, respectively; and wherein the blank was a blank control, CR was pAV-CAG-CYP4V2-P2A-RdCVF-BGH transfection group, and CspR was pAV-CAG-CYP4V2-P2A-spGL-RdCVF-BGH transfection group. It could be known from the results in Fig. 5 that after the signal peptide sequence was inserted into the RdCVF coding region, the expression of CYP4V2 was not affected, while the intracellular expression level of RdCVF was essentially unchanged and the extracellular secretion amount was significantly increased. It could be known that the signal peptide at the N-terminus of RdCVF could enhance the secretion effect of RdCVF without affecting the expressions of other proteins in the same CDS on the same vector.

### Example 6 GL signal peptide enhances the retinal ERG level of RdCVF in the Rd mouse model

After the AAV8 adeno-associated viruses AAV8-CAG-RdCVF (SEQ ID NO.13) and AAV8-CAG-spGL-RdCVF (SEQ ID No.14) carrying CAG-RdCVF or CAG-spGL-RdCVF nucleotide molecule were packaged and purified, 3-week-old RPGR mutant male mice (purchased from Biocytogen Pharmaceuticals (Beijing) Co., Ltd., RPGR ORF15 mutation) were subretinal injected, and observed for 3 months after injection.

Process for subretinal injection:
1) The experiment materials were prepared, the mice's pupils were dilated with 1% atropine, and then the mice were anesthetized by intraperitoneal injection of 80 mg/kg ketamine + 8 mg/kg xylazine.
2) After the anesthesia, the pupils were dilated with 1% atropine again. Then the mice were placed in front of the animal experiment platform of the ophthalmic surgery microscope (Topcon, OMS800), and 0.5% proparacaine was dropped on the eyeballs of mice for local anesthesia.
3) The fluorescein sodium stock solution was added to the virus at a concentration of fluorescein sodium : virus = 100:1, and mixed with a pipette.
4) A minipore was pricked by insulin needle in advance in the ciliary pars plana of the mouse eyeball, through which a microsyringe needle passed to enter the vitreous chamber of the mouse eyeball. At this time, an appropriate amount of 2% hydroxymethyl cellulose was dropped on the mouse eyeball such that the mouse fundus could be seen under the microscope. Then the needle was inserted into the contralateral periphery retina while keeping off the lens. The viruses with sodium fluorescein were slowly pushed-in, with an injection volume of 1 µl in each eye and a virus concentration of 1×10⁹. The fluorescein sodium served as the indicator for judging whether it was injected into the subretinal space.

After the operation, the surface of the eyeball was washed with normal saline and the mouse was placed in a cage to wait for waking up.

*In vivo* retinal function test: electroretinogram (ERG)
1) Dark adaptation of mice: the mice were subjected to the dark adaptation for at least 16 hours, after which all the operations were performed under dark red light.
2) Anesthesia of mice: the anesthesia was performed by intraperitoneal injection of 80 mg/kg ketamine + 8 mg/kg xylazine.
3) After the anesthesia was completed, the pupils in the eyes of mice were dilated with 1% atropine under the illumination of dark red light. The mice were fixed with adhesive tape in front of the animal experiment platform of the visual electrophysiology instrument Espion E2, and the eyes were consistent and fully exposed. The ground electrode needle was inserted into the root of the mouse tail, and the reference electrode needle was inserted into the mouse jaw. Two gold ring recording electrodes were clamped on the electrode holder of the animal experiment platform, and their angles were adjusted so that they slightly touched the top end in the center of the left and right corneas, respectively. An appropriate amount of 2% hydroxymethyl cellulose was dropped to improve the contact effect between the gold ring electrode and the cornea.
4) The information about mouse number and age was entered in the Espion E2 computer system and then the program was run. The dark-adaptation with a flash intensity of 0.003, 0.01, 0.1, 1, 3, 10, and 100 cd·s/m², respectively (the background light intensity was 0 cd·s/m², the stimulation interval was 15 s, and the average of three ERG signals was recorded) and the light-adaptation with a flash intensity of 3, 10, 30, and 100 cd·s/m², respectively (the light-adaptation time was 5 min, the background light intensity was 30 cd·s/m², the stimulation interval was 15 s, and the average of five ERG signals was recorded) were run. After the program was completed, the running results were automatically saved, and GraphPad Prism was used for the statistics of results.

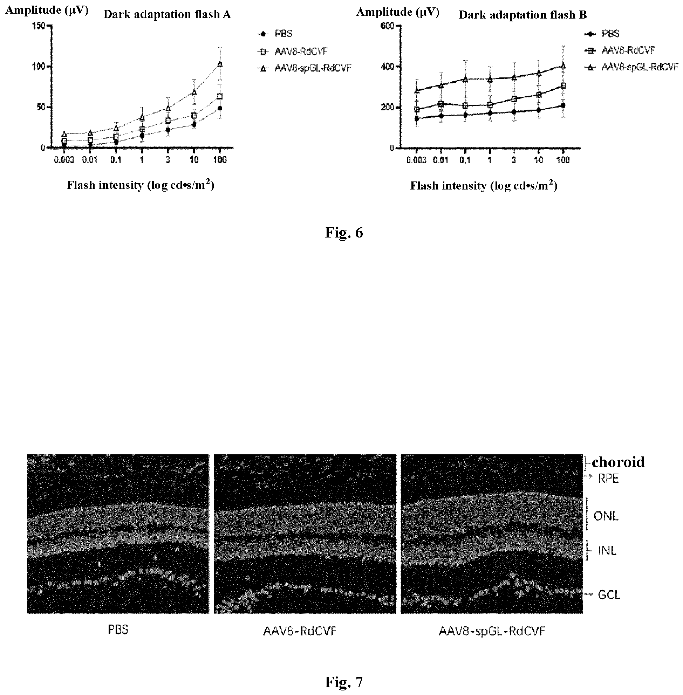

Fig. 6 showed the ERG record results for BCD mice after the treatments with various viruses through subretinal injection. The results showed that the ERG amplitudes for the mice in the groups injected with AAV8-CAG-RdCVF and AAV8-CAG-spGL-RdCVF were increased, and the virus group AAV8-CAG-spGL-RdCVF group was superior to the virus group AAV8-CAG-RdCVF.

### Example 7 GL signal peptide enhances the maintenance function of RdCVF on retinal photoreceptor cells in the Rd mouse model

The histomorphological observation on the retina of the mice in Example 6 was carried out:
1) Fixation and dehydration: the mice were sacrificed by cervical dislocation to remove the eyeballs. The mouse eyeball was placed in a 1.5 ml EP tube, and 1 ml of 4% paraformaldehyde was added at 4°C overnight. Then the eyeball was transferred into a 1.5 ml EP tube containing 1 ml of 30% sucrose solution for dehydration, until the eyeball sank to the bottom.
2) The mouse eyeball was placed into a 1.5 ml EP tube containing optimal cutting temperature compound (OCT). The mouse eye was positioned by tweezer to look straight ahead. The EP tube was capped and placed in liquid nitrogen. After being completely frozen, the frozen sections were obtained, with a section thickness of 7 µm. After fixation in acetone at 4°C for 10 min, they were stored at -80 °C.
3) The sections were taken out, and after returning to room temperature, washed with PBS for 3 times (each for 5 min).
4) The glass slide was washed with PBS for 3 times, and DAPI diluent (1:5000) was added for incubating at room temperature for 15 min.
5) After dropping the anti-fluorescence-quenching mounting medium, the glass slide was covered with a coverslip, and stored at -20°C in dark.

The Nikon fluorescence microscope was used for observation and imaging.

The immunofluorescence results were shown in Fig. 7. It could be known from Fig. 7 that compared with the control group injected with PBS, the lengths of the outer nuclear layer (ONL) and the inner nuclear layer (INL) in the groups injected with AAV8-CAG-RdCVF and AAV8-CAG-spGL-RdCVF could be increased, and the ONL and INL lengths in the AAV8-CAG-spGL-RdCVF group were larger than those in the AAV8-CAG-RdCVF group.

The foregoing detailed descriptions are provided by way of explanation and illustration, and are not intended to limit the scope of the appended claims. Various modifications to the embodiments presently listed in the present application will be apparent to those of ordinary skill in the art and remained within the scope of the appended claims and equivalents thereof.

## Claims

1. A fusion protein comprising RdCVF and a signal peptide, wherein the signal peptide is an amino acid sequence set forth in any one of SEQ ID NOs: 27-31 and 33.

2. The fusion protein according to claim 1, wherein the RdCVF is human RdCVF.

3. The fusion protein according to any one of claims 1-2, wherein the RdCVF comprises an amino acid sequence set forth in SEQ ID NO: 34.

4. The fusion protein according to any one of claims 1-3, wherein the C-terminus of the signal peptide is directly or indirectly linked to the N-terminus of the RdCVF.

5. The fusion protein according to any one of claims 1-4, further comprising a second protein different from the RdCVF.

6. The fusion protein according to claim 5, wherein the C-terminus of the second protein is directly or indirectly linked to the N-terminus of the signal peptide.

7. The fusion protein according to any one of claims 1-6, sequentially comprising, from N-terminus to C-terminus, the second protein, the signal peptide, and the RdCVF.

8. The fusion protein according to any one of claims 5-7, wherein the second protein comprises CYP4V2.

9. The fusion protein according to claim 8, wherein the CYP4V2 comprises an amino acid sequence set forth in SEQ ID NO: 35.

10. The fusion protein according to any one of claims 1-9, comprising an amino acid sequence set forth in any one of SEQ ID NOs: 48-52, 54, and 55.

11. An isolated nucleic acid molecule, comprising a) a polynucleotide encoding RdCVF and b) a polynucleotide encoding a signal peptide.

12. The isolated nucleic acid molecule according to claim 11, wherein the RdCVF is human RdCVF.

13. The isolated nucleic acid molecule according to any one of claims 11-12, wherein the RdCVF comprises an amino acid sequence set forth in SEQ ID NO: 34.

14. The isolated nucleic acid molecule according to any one of claims 11-13, wherein the polynucleotide encoding RdCVF comprises a nucleotide sequence set forth in SEQ ID NO: 22.

15. The isolated nucleic acid molecule according to any one of claims 11-14, wherein the polynucleotide encoding the signal peptide is located at 5' end of the polynucleotide encoding RdCVF and operably linked to the polynucleotide encoding RdCVF.

16. The isolated nucleic acid molecule according to any one of claims 11-15, wherein the 3' end of the polynucleotide encoding the signal peptide is linked to the 5' end of the polynucleotide encoding RdCVF.

17. The isolated nucleic acid molecule according to any one of claims 11-16, wherein the type of the signal peptide includes Sec/SPI, Sec/SPII, and/or Tat/SPI.

18. The isolated nucleic acid molecule according to any one of claims 11-17, wherein the signal peptide has at least 9 amino acids in length.

19. The isolated nucleic acid molecule according to any one of claims 11-18, wherein the signal peptide comprises an amino acid sequence set forth in any one of SEQ ID NOs: 27-31 and 33.

20. The isolated nucleic acid molecule according to any one of claims 11-19, wherein the polynucleotide encoding the signal peptide comprises an amino acid sequence set forth in any one of SEQ ID NOs: 15-19 and 21.

21. The isolated nucleic acid molecule according to any one of claims 11-20, comprising a promoter.

22. The isolated nucleic acid molecule according to claim 21, wherein the promoter is located at 5' end of the polynucleotide encoding RdCVF and operably linked to the polynucleotide encoding RdCVF.

23. The isolated nucleic acid molecule according to any one of claims 21-22, wherein the promoter is located at 5' end of the polynucleotide encoding the signal peptide and operably linked to the polynucleotide encoding the signal peptide.

24. The isolated nucleic acid molecule according to any one of claims 21-23, wherein the 3' end of the promoter is linked to the 5' end of the polynucleotide encoding the signal peptide.

25. The isolated nucleic acid molecule according to any one of claims 21-24, wherein the promoter comprises a nucleotide sequence set forth in SEQ ID NO: 23.

26. The isolated nucleic acid molecule according to any one of claims 11-25, comprising a PolyA signal site.

27. The isolated nucleic acid molecule according to claim 26, wherein the PolyA signal site is located at 3' end of the polynucleotide encoding RdCVF and operably linked to the polynucleotide encoding RdCVF.

28. The isolated nucleic acid molecule according to any one of claims 26-27, wherein the 3' end of the polynucleotide encoding RdCVF is linked to the 5' end of the PolyA signal site.

29. The isolated nucleic acid molecule according to any one of claims 26-28, wherein the PolyA signal site comprises a nucleotide sequence set forth in SEQ ID NO: 25.

30. The isolated nucleic acid molecule according to any one of claims 26-29, sequentially comprising, in 5' to 3' direction: the promoter, the polynucleotide encoding the signal peptide, the polynucleotide encoding RdCVF, and the PolyA signal site.

31. The isolated nucleic acid molecule according to any one of claims 21-30, comprising a second polynucleotide encoding a second protein, wherein the second protein is different from RdCVF.

32. The isolated nucleic acid molecule according to claim 31, wherein the second polynucleotide and the polynucleotide encoding RdCVF are located in the same coding sequence (CDS) region.

33. The isolated nucleic acid molecule according to claim 32, comprising a polynucleotide encoding a self-cleaving peptide.

34. The isolated nucleic acid molecule according to claim 33, wherein the polynucleotide encoding the self-cleaving peptide is located between the second polynucleotide and the polynucleotide encoding RdCVF.

35. The isolated nucleic acid molecule according to any one of claims 33-34, wherein the self-cleaving peptide comprises P2A.

36. The isolated nucleic acid molecule according to any one of claims 33-35, wherein the polynucleotide encoding the self-cleaving peptide comprises a nucleotide sequence set forth in SEQ ID NO: 26.

37. The isolated nucleic acid molecule according to any one of claims 33-36, sequentially comprising, in 5' to 3' direction: the promoter, the second polynucleotide, the polynucleotide encoding the self-cleaving peptide, the polynucleotide encoding the signal peptide, the polynucleotide encoding RdCVF, and the PolyA signal site.

38. The isolated nucleic acid molecule according to any one of claims 33-37, sequentially comprising, in 5' to 3' direction: the promoter, the polynucleotide encoding RdCVF, the polynucleotide encoding the self-cleaving peptide, the polynucleotide encoding the signal peptide, the second polynucleotide, and the PolyA signal site.

39. The isolated nucleic acid molecule according to claim 31, wherein the second polynucleotide and the polynucleotide encoding RdCVF are located in different protein coding sequence (CDS) regions.

40. The isolated nucleic acid molecule according to claim 39, comprising at least two promoters.

41. The isolated nucleic acid molecule according to any one of claims 39-40, comprising at least two PolyA signal sites.

42. The isolated nucleic acid molecule according to any one of claims 39-41, sequentially comprising, in 5' to 3' direction: the promoter, the second polynucleotide, the PolyA signal site, the promoter, the polynucleotide encoding the signal peptide, the polynucleotide encoding RdCVF, and the PolyA signal site.

43. The isolated nucleic acid molecule according to any one of claims 39-41, sequentially comprising, in 5' to 3' direction: the promoter, the polynucleotide encoding RdCVF, the PolyA signal site, the promoter, the polynucleotide encoding the signal peptide, the second polynucleotide, and the PolyA signal site.

44. The isolated nucleic acid molecule according to any one of claims 31-43, wherein the second protein comprises CYP4V2.

45. The isolated nucleic acid molecule according to any one of claims 31-44, wherein the second polynucleotide comprises a polynucleotide encoding CYP4V2.

46. The isolated nucleic acid molecule according to any one of claims 1-45, comprising a nucleotide sequence set forth in any one of SEQ ID NOs: 37-41, 43, 45, and 47.

47. A vector comprising the isolated nucleic acid molecule according to any one of claims 1-46.

48. The vector according to claim 47, comprising a viral vector.

49. The vector according to any one of claims 47-48, which is a viral vector, and the viral vector comprises an AAV vector.

50. A cell comprising the isolated nucleic acid molecule according to any one of claims 1-46 or the vector according to any one of claims 47-49.

51. A pharmaceutical composition comprising the isolated nucleic acid molecule according to any one of claims 1-46, the vector according to any one of claims 47-49, and/or the cell according to claim 50, as well as a pharmaceutically acceptable carrier.

52. Use of the isolated nucleic acid molecule according to any one of claims 1-46, the vector according to any one of claims 47-49, the cell according to claim 50, and/or the pharmaceutical composition in the manufacture of a medicament for treating, alleviating, and/or preventing a disease or disorder associated with retinal pigment epithelium (RPE) atrophy.

53. The use according to claim 52, wherein the disease or disorder comprises Bietti's crystalline dystrophy (BCD).

54. A method of increasing the expression level of RdCVF, comprising the step of making a nucleic acid molecule capable of encoding RdCVF to contain a polynucleotide encoding a signal peptide.

55. The method according to claim 54, comprising an *in vitro* method and/or an *in vivo* method.

56. The method according to any one of claims 54-55, wherein compared with a nucleic acid molecule without the polynucleotide encoding the signal peptide, the expression level of RdCVF secreted by a cell having the nucleic acid molecule comprising the polynucleotide encoding the signal peptide is significantly increased.

57. The method according to claim 56, wherein the cell comprises a mammalian cell.

58. The method according to any one of claims 54-57, wherein the RdCVF is human RdCVF.

59. The method according to any one of claims 54-58, wherein the RdCVF comprises an amino acid sequence set forth in SEQ ID NO: 34.

60. The method according to any one of claims 54-59, wherein the signal peptide comprises an amino acid sequence set forth in any one of SEQ ID NOs: 27-31 and 33.

61. The method according to any one of claims 54-60, wherein the nucleic acid molecule capable of encoding RdCVF comprises a polynucleotide encoding RdCVF, and the polynucleotide encoding RdCVF comprises a nucleotide sequence set forth in SEQ ID NO: 22.

62. The method according to any one of claims 54-61, wherein the polynucleotide encoding the signal peptide is located at 5' end of the nucleic acid molecule encoding RdCVF and operably linked to the polynucleotide encoding RdCVF in the nucleic acid molecule encoding RdCVF.

63. The method according to any one of claims 54-62, wherein the 3' end of the polynucleotide encoding the signal peptide is linked to the 5' end of the polynucleotide encoding RdCVF.

64. The method according to any one of claims 54-63, wherein the type of the signal peptide includes Sec/SPI, Sec/SPII, and/or Tat/SPI.

65. The method according to any one of claims 54-64, wherein the signal peptide has at least 9 amino acids in length.

66. The method according to any one of claims 54-65, wherein the polynucleotide encoding the signal peptide comprises an amino acid sequence set forth in any one of SEQ ID NOs: 15-19 and 21.

67. The method according to any one of claims 54-66, wherein the nucleic acid molecule capable of encoding RdCVF comprises a promoter.

68. The method according to claim 67, wherein the promoter is located at 5' end of the polynucleotide encoding RdCVF and operably linked to the polynucleotide encoding RdCVF.

69. The method according to any one of claims 67-68, wherein the promoter is located at 5' end of the polynucleotide encoding the signal peptide and operably linked to the polynucleotide encoding the signal peptide.

70. The method according to any one of claims 67-69, wherein the 3' end of the promoter is linked to the 5' end of the polynucleotide encoding the signal peptide.

71. The method according to any one of claims 67-70, wherein the promoter comprises a nucleotide sequence set forth in SEQ ID NO: 23.

72. The method according to any one of claims 54-71, wherein the nucleic acid molecule capable of encoding RdCVF comprises a PolyA signal site.

73. The method according to claim 72, wherein the PolyA signal site is located at 3' end of the polynucleotide encoding RdCVF and operably linked to the polynucleotide encoding RdCVF.

74. The method according to any one of claims 72-73, wherein the 3' end of the polynucleotide encoding RdCVF is linked to the 5' end of the PolyA signal site.

75. The method according to any one of claims 72-74, wherein the PolyA signal site comprises a nucleotide sequence set forth in SEQ ID NO: 25.

76. The method according to any one of claims 72-75, wherein the nucleic acid molecule capable of encoding RdCVF sequentially comprises, in 5' to 3' direction: the promoter, the polynucleotide encoding the signal peptide, the polynucleotide encoding RdCVF, and the PolyA signal site.

77. The method according to any one of claims 54-76, wherein the nucleic acid molecule capable of encoding RdCVF comprises a second polynucleotide encoding a second protein, wherein the second protein is different from RdCVF.

78. The method according to claim 77, wherein the second polynucleotide and the polynucleotide encoding RdCVF are located in the same coding sequence (CDS) region.

79. The method according to claim 78, wherein the nucleic acid molecule capable of encoding RdCVF comprises a polynucleotide encoding a self-cleaving peptide.

80. The method according to claim 79, wherein the polynucleotide encoding the self-cleaving peptide is located between the second polynucleotide and the polynucleotide encoding RdCVF.

81. The method according to any one of claims 79-80, wherein the self-cleaving peptide comprises P2A.

82. The method according to any one of claims 79-81, wherein the polynucleotide encoding the self-cleaving peptide comprises a nucleotide sequence set forth in SEQ ID NO: 26.

83. The method according to any one of claims 79-82, wherein the nucleic acid molecule capable of encoding RdCVF sequentially comprises, in 5' to 3' direction: the promoter, the second polynucleotide, the polynucleotide encoding the self-cleaving peptide, the polynucleotide encoding the signal peptide, the polynucleotide encoding RdCVF, and the PolyA signal site.

84. The method according to any one of claims 79-83, wherein the nucleic acid molecule capable of encoding RdCVF sequentially comprises, in 5' to 3' direction: the promoter, the polynucleotide encoding RdCVF, the polynucleotide encoding the self-cleaving peptide, the polynucleotide encoding the signal peptide, the second polynucleotide, and the PolyA signal site.

85. The method according to any one of claims 77-84, wherein the second polynucleotide and the polynucleotide encoding RdCVF are located in different coding sequence (CDS) regions.

86. The method according to claim 85, wherein the nucleic acid molecule capable of encoding RdCVF comprises at least two promoters.

87. The method according to any one of claims 85-86, wherein the nucleic acid molecule capable of encoding RdCVF comprises at least two PolyA signal sites.

88. The method according to any one of claims 85-87, wherein the nucleic acid molecule capable of encoding RdCVF sequentially comprises, in 5' to 3' direction: the promoter, the second polynucleotide, the PolyA signal site, the promoter, the polynucleotide encoding the signal peptide, the polynucleotide encoding RdCVF, and the PolyA signal site.

89. The method according to any one of claims 85-87, wherein the nucleic acid molecule capable of encoding RdCVF sequentially comprises, in 5' to 3' direction: the promoter, the polynucleotide encoding RdCVF, the PolyA signal site, the promoter, the polynucleotide encoding the signal peptide, the second polynucleotide, and the PolyA signal site.

90. The method according to any one of claims 77-89, wherein the second protein comprises CYP4V2.

91. The method according to claim 90, wherein the CYP4V2 comprises an amino acid sequence set forth in SEQ ID NO: 35.

92. The method according to any one of claims 77-91, wherein the second polynucleotide comprises a polynucleotide encoding CYP4V2.

93. The method according to any one of claims 90-93, wherein the nucleic acid molecule capable of encoding RdCVF comprises a nucleotide sequence set forth in SEQ ID NO: 24.
